# FASCICULE DE BREVET EUROPEEN

(11) **EP 1 713 406 B1**
(45) Date de publication et mention de la délivrance du brevet: **26.10.2016**
(21) Numéro de dépôt: 05717629.9
(22) Date de dépôt: 11.02.2005
(51) Int. Cl.: A61B 17/16

(54) **DISPOSITIF POUR LE SUIVI DE LA PENETRATION D' UN INSTRUMENT DANS UNE STRUCTURE ANATOMIQUE**
VORRICHTUNG ZUR ÜBERWACHUNG DES EINDRINGENS EINES INSTRUMENTS IN EINE ANATOMISCHE STRUKTUR
DEVICE FOR MONITORING THE PENETRATION OF AN INSTRUMENT INTO AN ANATOMICAL STRUCTURE

(30) Priorité: 11.02.2004 FR 0401362
(43) Date de publication de la demande: 25.10.2006
(73) Titulaire: SPINEGUARD, 94160 Saint Mandé (FR)
(72) Inventeur: BOURLION, Maurice, F-42400 Saint-Chamond (FR); VANACKER, Gérard, Tiburon , 94290 California (US); PETIT, Dominique, F-62180 Verton (FR)
(74) Mandataire: Cabinet Plasseraud
(86) Numéro de dépôt international: PCT/FR2005/000340
(87) Numéro de publication internationale: WO 2005/077283

(56) Documents cités:
- EP-A- 0 607 688
- WO-A-03/068076
- US-B1- 6 391 005

## Description

La présente invention concerne le domaine de la chirurgie rachidienne, et plus particulièrement le suivi des instruments de pénétration au cours des opérations de perçage vertébral, cervical, thoracique, lombaire, sacré ou ilio sacré.

L'art antérieur connaît déjà des dispositifs permettant le suivi de la pénétration d'un instrument dans une structure anatomique, en particulier une structure osseuse.

On connaît le brevet européen EP0607688 décrivant une procédure et un système d'insertion d'une vis vertébrale pédiculaire, consistant à appliquer un potentiel électrique à la surface de la cavité, et à observer les réactions musculaires provoquées par cette stimulation.

On connaît également une solution consistant à mesurer la modification d'impédance dans la région voisine de la cavité osseuse explorée, à l'aide d'une sonde présentant une électrode venant en contact avec la paroi de la cavité osseuse, et une deuxième électrode placée sur le patient. Le but est de détecter des brèches dans la matière osseuse, par exemple lors d'une opération de préparation de la pose d'une vis pédiculaire dans une vertèbre.

L'information recueillie avec une telle solution est difficile à interpréter, car l'impédance mesurée entre les deux électrodes est perturbée par des artefacts liés à la variation d'enfoncement de la sonde dans la cavité. Les résistivités de l'air, des tissus musculaires, des tissus osseux et des brèches sont différentes, et le signal mesuré est une résultante de plusieurs paramètres masquant en partie l'information utile correspondant au passage de l'électrode de la sonde à proximité d'une brèche.

En outre, le dispositif proposé reste peu pratique du fait qu'il est nécessaire d'effectuer préalablement un calibrage (référence liée aux tissus mous).

Enfin, un tel dispositif reste de manipulation peu aisée du fait de la présence de câblages externes.

Des instruments de pénétration selon le préambule de la revendication 1 sont décrits dans les documents WO 03/068076 et US 6 391 005

Le but de l'invention est de remédier à ces inconvénients en proposant un dispositif amélioré, dont le signal de sortie n'est pas perturbé par les variations dues à la profondeur d'engagement de l'instrument de pénétration.

La présente invention a également pour but de proposer un dispositif autonome, ne nécessitant aucun câblage externe.

La présente invention a également pour but de proposer un dispositif offrant des conditions de forage améliorées et sécurisées en avertissant l'opérateur de la formation de brèches.

À cet effet, l'invention concerne selon son acception la plus générale un dispositif pour le suivi de la pénétration d'un instrument dans une structure anatomique, en particulier une structure osseuse, comportant une source de tension alimentant au moins deux électrodes située sur ledit instrument et un moyen de mesure de l'impédance entre lesdites électrodes, et elle est remarquable en ce que lesdites électrodes sont situées sur ledit instrument de pénétration de façon à présenter une surface de contact affleurante et constante en fonction du degré d'enfoncement dudit instrument de pénétration dans ladite structure osseuse.

Plus précisément, la constance de la surface de contact des électrodes au cours de l'enfoncement dudit instrument de pénétration est obtenue de par les dimensions de ladite surface au regard des dimensions du trou formé dans la structure osseuse par ledit instrument de pénétration, ladite surface de contact devant présenter des dimensions inférieures à celles du trou formé par ledit instrument de pénétration.

Par la notion de « surface de contact », il doit donc être compris le fait que la surface affleurante des électrodes présente des dimensions inférieures aux dimensions du trou formé par ledit instrument de pénétration.

Ledit dispositif comporte une électrode affleurant la surface distale dudit instrument de pénétration.

Par surface distale, on entend la surface de l'extrémité distale dudit instrument de pénétration.

Selon une première variante de l'invention, ledit dispositif comporte deux électrodes affleurant la surface distale dudit instrument de pénétration, lesdites électrodes étant disposées coaxialement et séparées l'une de l'autre par un isolant.

Selon une variante de réalisation de l'invention, ledit dispositif comporte deux électrodes affleurant la surface distale dudit instrument de pénétration, lesdites électrodes étant disposées l'une par rapport à l'autre symétriquement par rapport à l'axe longitudinal dudit instrument de pénétration.

Selon une autre variante de réalisation de l'invention, ledit dispositif comporte une pluralité d'électrodes affleurant la surface distale dudit instrument de pénétration.

Ledit dispositif comporte une pluralité d'électrodes présentant une surface de contact affleurant latéralement ledit instrument de pénétration.

Ladite pluralité d'électrodes présente des surfaces de contact annulaire.

Ledit dispositif comporte une électrode principale affleurant la surface distale dudit instrument de pénétration ainsi qu'une pluralité d'électrodes secondaires affleurant latéralement pour former des contacts annulaires espacés longitudinalement.

Selon un mode de réalisation préféré de l'invention, ledit dispositif comporte en outre des moyens de signalisation produisant un signal lors de la détection d'une variation de l'impédance par ledit moyen de mesure.

Avantageusement, le signal produit est un signal sonore dont la fréquence et/ou la cadence diminue(nt) en fonction de l'impédance mesurée. De préférence, la fréquence et/ou la cadence diminue(nt) non linéairement en fonction de l'impédance mesurée.

Ainsi, lorsque ledit instrument sort de la structure osseuse, le signal produit est un signal sonore aiguë à cadence rapide ; lorsque ledit instrument pénètre et reste dans la structure osseuse, le signal produit est un signal sonore grave à faible cadence.

Avantageusement, ledit dispositif comporte un canal central pour le passage d'un instrument additionnel.

L'invention sera mieux comprise à la lecture de la description qui suit, se référant aux figures annexées où :
- les figures 1A et 1B illustrent respectivement une vue en coupe frontale et une vue en coupe longitudinale d'un instrument de forage constituant un dispositif d'exploration non selon l'invention ;
- la figure 2 illustre une vue en coupe frontale d'une première variante de réalisation de l'instrument de forage ;
- la figure 3 illustre une représentation graphique du signal sonore émis par le dispositif d'exploration en fonction de l'impédance mesurée ;
- la figure 4 illustre une vue en coupe longitudinale d'une réalisation de l'instrument de forage selon l'invention ;
- la figure 5 illustre une vue en perspective d'une variante de réalisation de l'instrument de forage ;
- la figure 6 illustre une vue en coupe longitudinale d'un instrument de pénétration constitué d'un taraud ; et
- la figure 7 illustre une vue en coupe longitudinale de l'instrument de pénétration selon une autre variation de l'instrument de forage.

Le dispositif selon l'invention est un dispositif permettant le suivi de la pénétration d'un instrument dans les structures osseuses d'un corps humain ou animal, lesdites structures présentant au moins deux zones d'impédance électrique différentes.

Lesdites électrodes, situées sur ledit instrument de pénétration (1), sont configurées pour présenter une surface de contact restant constante au cours de la pénétration dudit instrument de pénétration.

Lesdites électrodes sont reliées chacune à un générateur électrique délivrant une tension alternative, lequel comprend un circuit de mesure de l'impédance entre les deux électrodes (impédancemètre).

Ainsi, l'impédance des tissus pédiculaires étant strictement supérieure à celle des tissus musculaires, la détection d'une brèche se traduit par une diminution de l'impédance.

Ledit dispositif comporte en outre des moyens de signalisation produisant un signal spécifique lors de la détection, par l'impédancemètre, d'une variation d'impédance, et donc de la pénétration de l'instrument dans une zone de tissus mous (moelle, nerfs), pour former ainsi une brèche dans le cortex osseux. Lesdits moyens de signalisation consistent en l'émission d'un signal visuel, tel qu'un témoin lumineux, d'un signal sonore, et/ou d'un signal tactile (vibreur, ...).

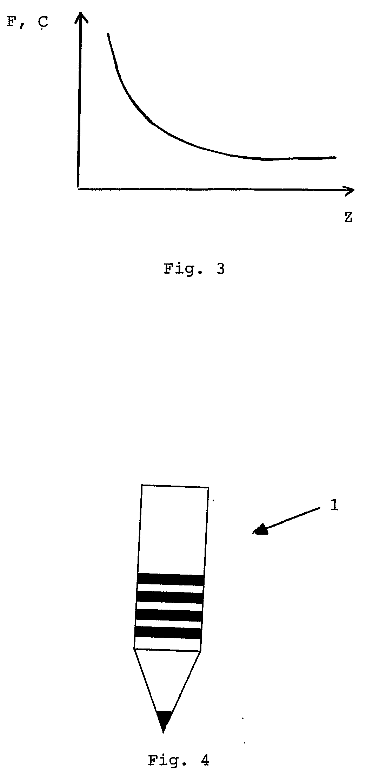

Un exemple préféré du principe de fonctionnement de la signalisation de la détection d'une brèche est décrit plus loin (figure 3).

Dans la partie ci-après, l'instrument de pénétration consiste en un instrument de forage (1). Cependant les configurations présentées ci-dessous sont bien entendu applicables aux autres instruments de pénétration (taraudage, curetage, spatulage, ...).

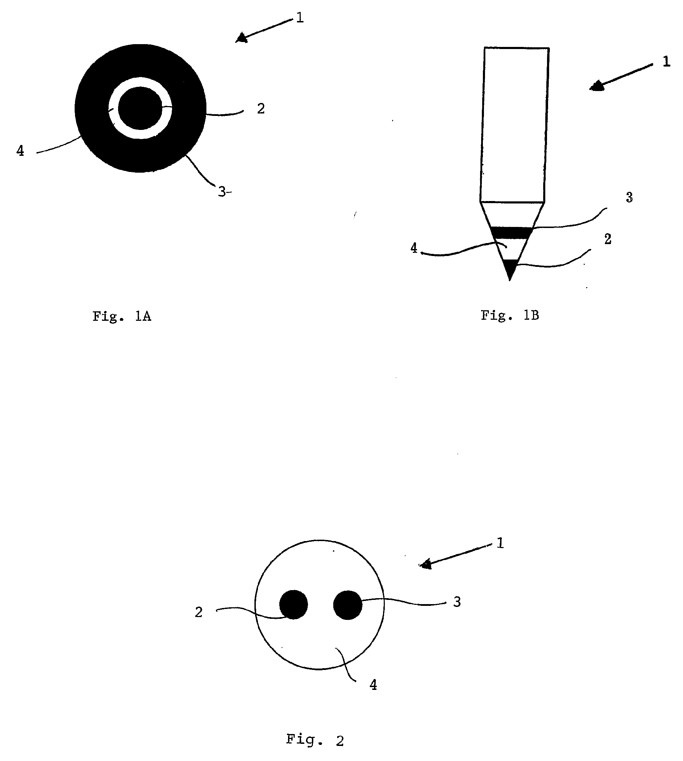

Les figures 1A et 1B illustrent une première configuration d'un instrument de forage (1).

Dans cette première configuration, l'instrument de forage (1) présente au niveau de son extrémité distale, deux électrodes (2, 3) de section circulaire et concentrique, l'électrode (2) intérieure étant séparée de l'électrode (3) extérieure par une couronne d'isolant (4).

L'électrode (2) constitue, dans cet exemple de réalisation, le pôle positif dudit dispositif électronique, l'électrode (3) le pôle négatif. Il est bien entendu évident qu'il ne s'agit ici que d'un exemple, et que l'homme du métier pourra réaliser un dispositif électronique dont le pôle positif sera constitué par l'électrode (3) et le pôle négatif par l'électrode (2).

Chaque électrode (2, 3) est disposée de sorte à affleurer la surface distale dudit instrument de forage (1).

Afin d'éviter toute perturbation du signal, la surface de l'électrode (3) affleurant la surface dudit instrument de forage (1) reste relativement petite par rapport aux dimensions du trou effectué dans le cortex osseux lors de l'opération de forage.

Lors de la pénétration de l'instrument (1) dans la structure osseuse, un signal est émis par lesdits moyens de signalisation lorsqu'une variation d'impédance mesurée entre lesdites électrodes (2, 3) est détectée par l'impédancemètre, indiquant la formation d'une brèche

A cet instant, le praticien est informé que l'extrémité de l'instrument de forage (1) vient de sortir du cortex osseux pour pénétrer dans une zone de tissus mous. Le praticien, s'il le souhaite, modifie alors la trajectoire de l'instrument de forage (1) de sorte à revenir dans le cortex osseux.

La figure 2 illustre une seconde configuration de l'instrument de forage (1).

Dans cette seconde configuration, l'instrument de pénétration (1) présente au niveau de son extrémité distale deux électrodes (2, 3) de section circulaire sensiblement identique. Lesdites électrodes (2, 3) sont avantageusement disposées symétriquement par rapport à l'axe longitudinal de l'instrument de forage (1).

La position desdites électrodes (2, 3) étant connue, leur disposition sur l'extrémité distale donne des indications sur la position des brèches. En effet, la brèche détectée sera située entre les deux électrodes (2, 3) pour lesquelles un signal est émis.

Le nombre et la forme des électrodes étant donné ici à titre d'exemple, il est entendu que ledit instrument (1) de pénétration peut présenter des électrodes en nombre supérieur et de forme différente. Il est à noter que la détection volumétrique de brèches sera d'autant plus précise que le nombre d'électrodes réparties à l'extrémité dudit instrument (1) sera élevé.

La figure 3 illustre la représentation graphique de la fréquence et/ou cadence d'un signal sonore émis par lesdits moyens de signalisation en fonction de l'impédance mesurée entre les électrodes.

Selon un mode de réalisation préférentiel de l'invention, la courbe correspondant à la fréquence et/ou la cadence du signal émis en fonction de l'impédance est décroissante et non linéaire (cf. figure 3). Ainsi, lorsque l'instrument de pénétration est situé dans le cortex osseux, l'impédance mesurée entre les électrodes correspond à l'impédance de l'os, cette impédance restant relativement constante. Lesdits moyens de signalisation informent le praticien de la position correcte dans le cortex par l'émission d'un signal de fréquence grave et de cadence lente. En particulier, au-delà d'une certaine valeur de l'impédance, correspondant à l'impédance mesurée dans l'os, la fréquence ainsi que la cadence des signaux restent relativement constantes.

En revanche, lorsque l'extrémité de l'instrument pénètre dans un tissu environnant mou, le praticien en est averti par une augmentation de la fréquence et une accélération de la cadence du signal.

Ainsi, suivant cette configuration, une faible variation de l'impédance dans l'os ne s'entendra pas alors que, toute variation d'impédance liée à la pénétration de l'instrument dans un tissu environnant mou, aussi faible soit elle, s'entendra fortement.

De la même façon, il est possible de réaliser des instruments de pénétration présentant d'autres fonctionnalités.

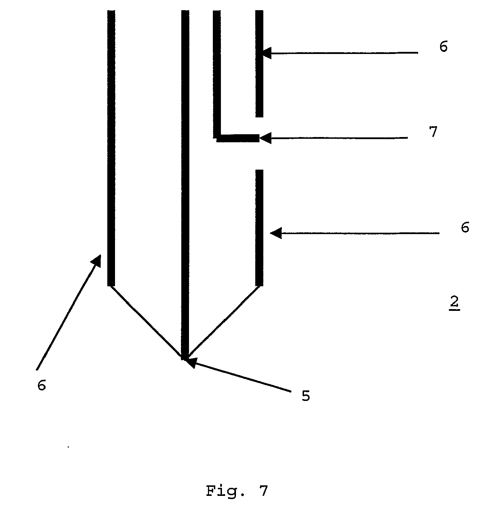

En particulier, ledit instrument (1) de forage pourra avantageusement comporter au moins une électrode (7) affleurant la surface latérale dudit instrument (1) de forage, ainsi que deux électrodes (5, 6) disposées concentriquement à l'extrémité distale dudit instrument (1) de forage (figure 7). Il sera ainsi possible, de par la configuration dudit instrument (1) de forage de déterminer la présence et la direction d'une brèche au moyen des électrodes (6, 7), ainsi que de prévenir une éventuelle perforation du cortex osseux au moyen des électrodes (5, 6). A cet effet, il devra être évité de positionner une électrode latérale consistant en une tige allant jusqu'à l'extrémité distale. Il serait en effet impossible, avec une telle configuration, de savoir si la zone détectée par les électrodes est latérale ou distale.

Selon l'invention, des électrodes sont disposées sur la surface latérale de l'instrument de forage pour former des bandes de contact annulaires affleurant la surface de l'instrument de forage (1) (figure 4).

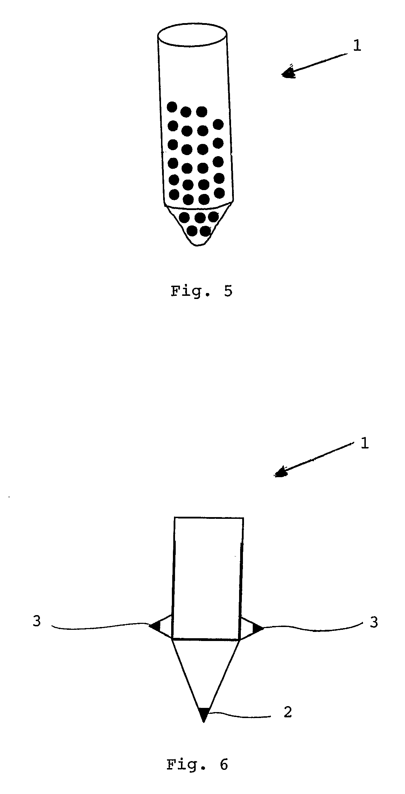

Selon une variante de réalisation non selon l'invention, les électrodes seront avantageusement disposées sous la forme de points de contact répartis de façon homogène sur la surface de l'instrument de forage (1), une telle répartition des électrodes permettant une détection volumétrique des perforations (figure 5). Une telle configuration permet ainsi d'informer à chaque instant le chirurgien de la zone d'impédance la plus faible.

La figure 6 illustre également la réalisation d'un instrument de pénétration configuré pour le taraudage, avec deux électrodes (10, 11) disposées concentriquement à l'extrémité distale dudit instrument (2) de pénétration (figure 6). Il sera ainsi possible, de part la configuration dudit instrument (2) de pénétration de déterminer la présence et la direction d'une brèche au moyen des électrodes (11 et 13), ainsi que de prévenir une éventuelle perforation du cortex osseux au moyen des électrodes (10 et 11). A cet effet, il devra être évité de positionner une électrode latérale consistant en une tige allant jusqu'à l'extrémité distale. Il serait en effet impossible, avec une telle configuration, de savoir si la zone détectée par les électrodes est latérale ou distale.

L'invention est décrite dans ce qui précède à titre d'exemple. Il est entendu que l'homme du métier est à même de réaliser différentes variantes de l'invention sans pour autant sortir du cadre du brevet qui est defini par les revendications.

## Revendications

1. Instrument de pénétration dans une structure anatomique, en particulier une structure osseuse, présentant une surface latérale cylindrique selon un axe longitudinal, et une extrémité distale qui présente une surface distale conique selon l'axe longitudinal, l'instrument de pénétration comportant un dispositif pour le suivi de la pénétration de l'instrument de pénétration dans la structure anatomique, le dispositif comportant :
- au moins deux électrodes portées par l'instrument de pénétration,
- une source de tension alimentant lesdites électrodes et
- un moyen de mesure de l'impédance entre lesdites électrodes,
dans lequel lesdites au moins deux électrodes comportent au moins une première électrode présentant une surface de contact affleurant une surface distale dudit instrument de pénétration, et au moins une seconde électrode présentant une surface de contact, ladite au moins une première électrode comporte une électrode principale affleurant la surface distale dudit instrument de pénétration, lesdites surfaces de contact des première et seconde électrodes étant dimensionnées de sorte à présenter une surface restant constante au cours de l'enfoncement dudit instrument de pénétration dans ladite structure anatomique,
le dispositif étant **caractérisé en ce que** la surface de contact de ladite au moins une seconde électrode affleure la surface latérale de l'instrument de pénétration, et **en ce que** ladite au moins une seconde électrode comporte une pluralité d'électrodes secondaires affleurant la surface latérale de l'instrument de pénétration pour former des contacts annulaires espacés longitudinalement.

2. Instrument de pénétration selon l'une quelconque des revendications précédentes, **caractérisé en ce que** le dispositif comporte en outre des moyens de signalisation produisant un signal lors de la détection par ledit moyen de mesure de l'impédance une variation de l'impédance.

3. Instrument de pénétration selon la revendication précédente, **caractérisé en ce que** le signal produit est un signal sonore dont la fréquence et/ou la cadence diminue(nt) en fonction de l'impédance mesurée.

4. Instrument de pénétration selon la revendication précédente, **caractérisé en ce que** la fréquence et/ou la cadence diminue(nt) non linéairement en fonction de l'impédance mesurée.

5. Instrument de pénétration selon l'une quelconque des revendications 2 à 4, **caractérisé en ce que** le signal produit lorsque ledit instrument sort de la structure osseuse est un signal sonore aiguë à cadence rapide.

6. Instrument de pénétration selon l'une quelconque des revendications 2 à 5, **caractérisé en ce que** le signal produit lorsque ledit instrument pénètre la structure osseuse est un signal sonore grave à faible cadence.

7. Instrument de pénétration selon l'une quelconque des revendications précédentes, **caractérisé en ce que** ledit dispositif est un dispositif autonome.

8. Instrument de pénétration selon l'une quelconque des revendications précédentes, **caractérisé en ce qu'**il comporte un canal central pour le passage d'un instrument additionnel.

## Patentansprüche

1. Eindringungsinstrument zum Eindringen in eine anatomische Struktur, insbesondere eine Knochenstruktur, wobei das Eindringungsinstrument eine um eine Längsachse zylindrische Seitenfläche und ein distales Ende aufweist, das eine um die Längsachse konische distale Oberfläche hat, wobei das Eindringungsinstrument eine Vorrichtung zum Überwachen des Eindringens des Eindringungsinstruments in die anatomische Struktur aufweist, wobei die Vorrichtung aufweist:
- mindestens zwei von dem Eindringungsinstrument getragene Elektroden,
- eine die Elektroden versorgende Spannungsquelle und
- eine Einrichtung zum Messen der Impedanz zwischen den Elektroden,
wobei die mindestens zwei Elektroden mindestens eine erste Elektrode mit einer Kontaktfläche, die mit einer distalen Oberfläche des Eindringungsinstruments bündig ist, und mindestens eine zweite Elektrode mit einer Kontaktfläche aufweisen, wobei die mindestens eine erste Elektrode eine Hauptelektrode aufweist, die mit der distalen Oberfläche des Eindringungsinstruments bündig ist, wobei die Kontaktflächen der ersten und zweiten Elektrode(n) derart dimensioniert sind, dass sie eine Oberfläche aufweisen, die im Verlaufe des Hineintreibens des Eindringungsinstruments in die anatomische Struktur konstant bleibt,
wobei die Vorrichtung **dadurch gekennzeichnet ist, dass** die Kontaktfläche der mindestens einen zweiten Elektrode mit der Seitenfläche des Eindringungsinstruments bündig ist, und dadurch, dass die mindestens eine zweite Elektrode mehrere mit der Seitenfläche des Eindringungsinstruments bündige sekundäre Elektroden aufweist, um in Längsrichtung beabstandete ringförmige Kontakte zu bilden.

2. Eindringungsinstrument nach einem der vorstehenden Ansprüche, **dadurch gekennzeichnet, dass** die Vorrichtung desweiteren Signalisierungseinrichtungen aufweist, die ein Signal erzeugen, wenn von den Impedanzmesseinrichtungen eine Änderung der Impedanz detektiert wird.

3. Eindringungsinstrument nach dem vorstehenden Anspruch, **dadurch gekennzeichnet, dass** das erzeugte Signal ein akustisches Signal ist, dessen Schwingungsfrequenz und/oder Taktfrequenz sich in Abhängigkeit von der gemessenen Impedanz verringert/verringern.

4. Eindringungsinstrument nach dem vorstehenden Anspruch, **dadurch gekennzeichnet, dass** die Schwingungsfrequenz und/oder Taktfrequenz sich in Abhängigkeit von der gemessenen Impedanz nichtlinear verringert/verringern.

5. Eindringungsinstrument nach einem der Ansprüche 2 bis 4, **dadurch gekennzeichnet, dass** das Signal, das erzeugt wird, wenn sich das Instrument aus der Knochenstruktur heraus bewegt, ein schrilles Tonsignal mit hoher Taktfrequenz ist.

6. Eindringungsinstrument nach einem der Ansprüche 2 bis 5, **dadurch gekennzeichnet, dass** das Signal, das erzeugt wird, wenn sich das Instrument durch die Knochenstruktur bewegt, ein tiefes Tonsignal mit niedriger Taktfrequenz ist.

7. Eindringungsinstrument nach einem der vorstehenden Ansprüche, **dadurch gekennzeichnet, dass** die Vorrichtung eine unabhängige Vorrichtung ist.

8. Eindringungsinstrument nach einem der vorstehenden Ansprüche, **dadurch gekennzeichnet, dass** es einen zentralen Kanal zum Hindurchführen eines zusätzlichen Instruments aufweist.

## Claims

1. Instrument for penetrating an anatomical structure, in particular a bone structure, having a cylindrical lateral surface along a longitudinal axis and a distal end which has a conical distal surface along the longitudinal axis, the penetration instrument comprising a device for monitoring penetration of the penetration instrument in the anatomical structure, the device comprising:
- at least two electrodes borne by the penetration instrument,
- a voltage source supplying said electrodes and
- a means for measuring the impedance between said electrodes, wherein said at least two electrodes comprise at least one first electrode having a contact surface flush with a distal surface of said penetration instrument and at least one second electrode having a contact surface, said at least one first electrode comprises a main electrode flush with the distal surface of said penetration instrument, said contact surfaces of the first and second electrodes being dimensioned so as to have a surface that remains constant as said penetration instrument penetrates said anatomical structure,
the device being **characterised in that** the contact surface of said at least one second electrode is flush with the lateral surface of the penetration instrument and **in that** said at least one second electrode comprises a plurality of secondary electrodes flush with the lateral surface of the penetration instrument to form longitudinally spaced annular contacts.

2. Penetration instrument as claimed in any one of the preceding claims, **characterised in that** the device further comprises signalling means generating a signal when a change in impedance is detected by said impedance measuring means.

3. Penetration instrument as claimed in the preceding claim, **characterised in that** the generated signal is a sound signal, the frequency and/or rate of which decrease(s) as a function of the measured impedance.

4. Penetration instrument as claimed in the preceding claim, **characterised in that** the frequency and/or rate decrease(s) non-linearly as a function of the measured impedance.

5. Penetration instrument as claimed in any one of claims 2 to 4, **characterised in that** the signal generated when said instrument leaves the bone structure is a high-pitched sound signal with a fast rate.

6. Penetration instrument as claimed in any one of claims 2 to 5, **characterised in that** the signal generated when said instrument penetrates the bone structure is a low-pitched sound signal with a slow rate.

7. Penetration instrument as claimed in any one of the preceding claims, **characterised in that** said device is an autonomous device.

8. Penetration instrument as claimed in any one of the preceding claims, **characterised in that** it comprises a central channel providing a passage for an additional instrument.
